# EUROPEAN PATENT SPECIFICATION

(11) **EP 2 001 451 B1**
(45) Date of publication and mention of the grant of the patent: **11.08.2010**
(21) Application number: 07759471.1
(22) Date of filing: 27.03.2007
(51) Int. Cl.: A61K 9/40, A61K 9/42, A61K 9/32

(54) **NON-HOMOGENOUS DOSAGE FORM COATINGS**
FORMBESCHICHTUNGEN MIT UNGLEICHMÄSSIGER DOSIERUNG
ENROBAGES DE FORMES PHARMACEUTIQUES NON HOMOGENES

(30) Priority: 28.03.2006 US 743836 P
(43) Date of publication of application: 17.12.2008
(73) Proprietor: Mcneil-PPC, Inc, Skillman, NJ 08558 (US)
(72) Inventor: BUNICK, Frank J., Randolph, NJ 07869 (US); CHEN, Jen-chi, Morrisville, PA 19067 (US)
(74) Representative: Kirsch, Susan Edith
(86) International application number: PCT/US2007/065029
(87) International publication number: WO 2007/112395

(56) References cited:
- EP-A- 1 614 413
- WO-A-03/028619
- WO-A-2004/028514
- WO-A-2006/024479
- WO-A-2006/047695
- US-A- 4 629 619

## Description

The present invention relates to coating for pharmaceutical dosage forms wherein the coatings form at least a continuous shell portion having a first composition and at least one discontinuous shell portion that is compositionally different and located within the continuous shell portion. The coatings are preferably produced in an injection molding system.

### Background of the Invention

Various oral dosage forms have been developed over the years for pharmaceuticals and dietary supplements. Among the more popular oral dosage forms are tablets, capsules and, most recently, gelcaps. Tablets are compressed or molded solid dosage forms of any size or shape. Solid, generally oblong-shaped tablets may sometimes be referred to as caplets. Tablets remain popular with consumers, however uncoated tablets suffer from drawbacks such as medicinal taste, a tendency to powder or flake (i.e., physical disintegration) when packaged in bottles, and/or the perception by consumers that they are not easy to swallow. These limitations can be eliminated by coating the tablets with a polymeric coating.

During most of the 20th century, hard gelatin capsules were a popular dosage form for prescription and over-the-counter (OTC) drugs. Capsules are hard shell compartments made of two halves, including a body and a cap, wherein the cap partially and snugly overlaps with the body to enclose a dosable drug ingredient therein. The enclosed dosable ingredient is most often is a powder, liquid, paste or similar nonsolid form.

Generally, empty hard shell capsules are produced by a conventional dip-molding process such as that which is described on page 182 of "Pharmaceutical Dosage Forms and Drug Delivery Systems, 7th Ed.", (1999) by Howard C. Ansel, Loyd V. Allen Jr., and Nicholas G. Popovich, published by Lippincott Williams & Wilkins, Baltimore, Md. Consumers have found that such capsules are aesthetically pleasing, easy to swallow and mask the medicine taste of the drug contained therein. In addition, the bodies and caps of such capsules are often produced in different colors, resulting in a bi-colored capsule product having enhanced aesthetic appeal, as well as improved product identification and brand recognition by consumers. Many patients preferred capsules over coated or uncoated tablets, prompting pharmaceutical manufacturers to market certain products in capsule form even when they were also available in tablet form. However, due to potential tampering concerns, capsules are no longer a preferred delivery choice for consumer (i.e., over-the-counter) pharmaceuticals.

One alternative to capsule products are caplets, which are solid, oblong tablets that are often coated with various polymers such as cellulose ethers to improve their aesthetics, stability, and swallowability. Typically, such polymers are applied to the tablets either from solution in organic solvents, or from aqueous dispersion via spraying. Still other methods involve spray coating tablets with a gelatin coating solution. See, e.g., U.S. Pat. Nos. 4,973,480 and 6,113,945. However, such spray-coated tablets lack the glossy surface and elegance of the hard gelatin capsules. Additionally, it is not commercially feasible to spray-coat a tablet with a different color coating on each end.

Another alternative to capsule products are "gelcaps," which are elegant, consumer-preferred dosage forms comprising solid tablets covered with a glossy gelatinous coating. Currently, gelcaps are among the most popular oral dosage forms. Several methods of producing gelcaps have been developed in an effort to provide tamper-proof capsule-like products. One category of such methods involve dipping tablets, one half at a time, into gelatin coating solutions, which can be of two different colors, see, e.g., U.S. Pat. No. 4,820,524, or dipping tablets of a first color halfway into a into gelatin coating solution of a second color, see, e.g., U.S. Pat. No. 6,113,945. Another category of such methods involves shrink-fitting the capsule halves onto a tablet form. See, for example, U.S. Pat. Nos. 5,415,868, 6,126,767, 5,464,631, 5,460,824, 5,317,849, 5,511,361, 5,609,010, 5,795,588 and 6,080,426, and International Patent Appln. Publication No. WO 97/37629. Another method involves sealing the body and cap of the capsule at the overlapping seam therebetween. See U.S. Pat. No. 5,824,338. Another method of producing gelcaps is via an enrobing process wherein two separate films made of gelatinous material are applied to opposite sides of a tablet by a pair of rotary dies. A detailed description of this process is provided, for example, in U.S. Pat. Nos. 5,146,730 and 5,459,983.

### Summary of the Invention

The invention provides a liquid pharmaceutical dosage coating composition comprising:
a) a shell-forming component comprising a dispersion of:
   i) at least one film-forming polymer in liquid form that is liquid at temperatures of 37°C or above; and
   ii) at least one particle forming polymer in solid particle form for a discontinuous phase that is dispersed in said polymer in liquid form in (i),
wherein the solid particles have a number average particle size of at least 100 microns and wherein the solid particles are a low-moisture hydrocolloid solution with a water content up to 30%.

The present invention also provides a solid pharmaceutical dosage form comprising a core and at least one coating layer, which is formed from the liquid pharmaceutical dosage coating composition according to the invention, that comprises a solid continuous film having discrete compositionally different regions dispersed therein.. The coatings are preferably produced on the dosage form using an injection molding system.

The present invention also provides a process for preparing a solid dosage form, which comprises coating a core containing a pharmaceutical active ingredient with the composition according to the present invention.

In addition, the present invention provides a process for preparing a core and shell dosage form comprising:
a) forming a compressed core containing at least one pharmaceutical active ingredient in compression tableting machine; and
b) coating the compressed core with the composition according to the present invention.

The present invention further provides a process for preparing a core and shell dosage form comprising
a) forming a solid, compressed core containing at least one pharmaceutical active ingredient in a tableting machine;
b) introducing the compressed core into a mold cavity; and
c) injecting the composition according to the present invention into the mold cavity to coat at least a portion of the compressed core;
   and optionally comprising the steps of
d) rotating the mold cavity; and
e) injecting a liquid curable composition into said mold to coat at least a second portion of the compressed core.

### Detailed Description of the Invention

As used herein, the term "dosage form" applies to any solid object or semi-solid composition designed to contain a specific pre-determined amount (dose) of a certain ingredient, for example an active ingredient as defined below. Suitable dosage forms may be pharmaceutical drug delivery systems, including those for oral administration, buccal administration, rectal administration, or compositions for delivering minerals, vitamins and other nutraceuticals, oral care agents, flavorants, and the like. The dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human.

The core may be any solid form. The core can be prepared by any suitable method, including for example compression or molding. As used herein, "core" refers to a material which is at least partially enveloped or surrounded by another material over its exterior surface region. Preferably, the core is a self-contained unitary object, such as a tablet or capsule. Typically, the core comprises a solid, for example, the core may be a compressed or molded tablet, hard or soft capsule, suppository, or a confectionery form such as a lozenge, nougat, caramel, fondant, or fat based composition.

In certain other embodiments, the core or a portion thereof may be in the form of a semi-solid or a liquid in the finished dosage form. For example the core may comprise a liquid filled capsule, or a semisolid fondant material. In embodiments in which the core comprises a flowable component, such as a plurality of granules or particles, or a liquid, the core preferably additionally comprises an enveloping component, such as a capsule shell, or a coating, for containing the flowable material. In certain particular embodiments in which the core comprises an enveloping component, the shell or shell portions of the present invention are in direct contact with the enveloping component of the core, which separates the shell from the flowable component of the core.

Suitable active ingredients for use in this invention include for example pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, oral contraceptives, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

In one embodiment, the active ingredient is selected from analgesics, antiinflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefanamic acid, meclofenamic acid, flufenamic acid, and the like; biphenylcarbodylic acid derivatives, e.g. diflunisal, flufenisal, and the like; and oxicams, e.g. piroxicam, sudoxicam, isoxicam, meloxicam, and the like. In one particular embodiment, the active ingredient is selected from propionic acid derivative NSAID, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another particular embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from phenylephrine, pseudoephedrine, phenylpropanolamine, chlorpheniramine, dextromethorphan, diphenhydramine, astemizole, terfenadine, fexofenadine, loratadine, desloratadine, guaifenesin, sidenefil, chlorphedianol, menthol, benzocaine, modafinil, cetirizine, mixtures thereof and pharmaceutically acceptable salts, esters, isomers, and mixtures thereof.

The active ingredient or ingredients are present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dosing regimen, the age and weight of the patient, and other factors must be considered, as known in the art. Typically, the dosage form comprises at least about 1 weight percent, preferably, the dosage form comprises at least about 5 weight percent, e.g. at least about 25 weight percent of a combination of one or more active ingredients. In one embodiment, a core comprises a total of at least about 50 weight percent, e.g. at least about 70 weight percent, say at least about 80 weight percent (based on the weight of the core) of one or more active ingredients.

The active ingredient or ingredients may be present in the dosage form in any form. For example, the active ingredient may be dispersed at the molecular level, e.g. melted or dissolved, within the dosage form, or may be in the form of particles, which in turn may be coated or uncoated. If the active ingredient is in form of particles, the particles (whether coated or uncoated) typically have an average particle size of about 1-2000 microns. In one embodiment, such particles are crystals having an average particle size of about 1-300 microns. In another embodiment, the particles are granules or pellets having an average particle size of about 30-3000 microns, for example about 50-1000 microns, say about 100-800 microns.

In another embodiment the active ingredient is coated in the form of particles for the purposes of taste-masking or modified release. Modified release includes extended, sustained, controlled and enteric release. If the active ingredient has an objectionable taste, and the dosage form is intended to be chewed or disintegrated in the mouth prior to swallowing, the active ingredient may be coated with a taste masking coating, as known in the art. Examples of suitable taste masking coatings are described in, for example, U.S. Patent Nos. 4,851,226; 5,075,114; and 5,489,436. Commercially available taste masked active ingredients may also be employed. For example, acetaminophen particles, which are encapsulated with ethylcellulose or other polymers by a coacervation process, may be used in the present invention. Such coacervation-encapsulated acetaminophen is commercially available from Eurand America, Inc. or Circa Inc. Additional suitable processes for applying taste-masked coatings known in the art include but are not limited to fluid bed coating, coacervation, complex coacervation, spray drying and spray congealing.

The core may contain conventional ingredients such as fillers, including water soluble compressible carbohydrates such as sucrose, mannitol, sorbitol, maltitol, xylitol, erythritol, lactose, and mixtures thereof; conventional dry binders including cellulose, cellulosic derivatives, polyvinyl pyrrolidone, starch, modified starch, and mixtures thereof, and in particular microcrystalline cellulose; sweeteners including aspartame, acesulfame potassium, sucralose and saccharin; disintegrants such as microcrystalline cellulose, starch, sodium starch glycolate, crosslinked polyvinylpyrrolidone, crosslinked carboxymethylcellulose; and lubricants, such as magnesium stearate, stearic acid, talc, and waxes. The tablet may also incorporate pharmaceutically acceptable adjuvants, including for example preservatives, flavors, acidulants, antioxidants, glidants, surfactants, and coloring agents. Typically the total amount of these other conventional ingredients will not exceed about 25 percent of the tablet weight, i.e. not more than about 20 percent of the tablet weight, or not more than about 15 percent of the tablet weight.

Tablets of the present invention may be made by any means known in the art. Conventional methods for tablet production include direct compression ("dry blending"), dry granulation followed by compression, and wet granulation followed by drying and compression. Other methods include the use of compacting roller technology such as a chilsonator or drop roller, or molding, casting, or extrusion technologies. All of these methods are known in the art, and are described in detail in, for example, Lachman, et al., "The Theory and Practice of Industrial Pharmacy," Chapter 11, (3rd Ed. 1986).

In another embodiment, the core may be prepared by the compression methods and apparatus described in United States Patent Application Publication No. 20040156902. Specifically, the core may be made using a rotary compression module comprising a fill zone, insertion zone, compression zone, ejection zone, and purge zone in a single apparatus having a double row die construction as shown in Figure 6 of United States Patent Application Publication No. 20040156902. The dies of the compression module may then be filled using the assistance of a vacuum, with filters located in or near each die. The purge zone of the compression module includes an optional powder recovery system to recover excess powder from the filters and return the powder to the dies.

In another embodiment, the core may be prepared by a wet-granulation method, in which the active ingredient or ingredients, appropriate excipients, and a solution or dispersion of a wet binder (e.g. an aqueous cooked starch paste, or solution of polyvinyl pyrrolidone) may be mixed and granulated. Suitable apparatus for wet granulation include low shear, e.g. planetary mixers, high shear mixers, and fluid beds, including rotary fluid beds. The resulting granulated material may then be dried, and optionally dry-blended with further ingredients, e.g. adjuvants and/or excipients such as, for example, lubricants, colorants, and the like. The final dry blend is then suitable for compression by the methods described in the previous paragraph.

The core may be in a variety of different shapes. For example, in one embodiment the core may be in the shape of a truncated cone. In other embodiments the core may be shaped as a polyhedron, such as a cube, pyramid, prism, or the like; or may have the geometry of a space figure with some non-flat faces, such as a cone, cylinder, sphere, torus, or the like. Exemplary core shapes which may be employed include tablet shapes formed from compression tooling shapes described by "The Elizabeth Companies Tablet Design Training Manual" (Elizabeth Carbide Die Co., Inc., p.7 (McKeesport, Pa.) as follows (the tablet shape corresponds inversely to the shape of the compression tooling).

One or more outer coatings are applied over the core to form a shell. A coating differs from a compressed powder layer typically in terms of the degree of porosity, density and thickness. A compressed powder layer will generally have a higher degree of porosity, thickness and lower density.

The present invention is a dosage form having a coating that is provided onto one or more cores from a blend of materials wherein a first component of the blends flows in liquid form at temperatures equal to 37°C and above and a second component that is a solid particle and capable of being suspended within the first component in liquid form. In one embodiment the second component is solid at temperatures of at least 37°C. A liquid is a form of matter between a gas and a solid that has definite volume but no definite shape. For purposes of this application, the individual particles are considered solid as each particle has a definite shape. Further, a collection of such particles, while capable of flowing, would not be considered to be a liquid due to the tendency of the particles to separate and their lack of homogenous appearance under normal flow conditions found in pharmaceutical coating operations. A blend of immiscible liquids would still be considered "liquids" notwithstanding their capacity to separate due to the fact that neither of the individual component tends to separate or disassociate under normal flow conditions.

Upon drying, the dosage form will have a coating characterized by having a substantially smooth continuous coating with particles dispersed therein. The particles may or may not be evenly dispersed the continuous coating. A continuous coating means that the coating forms interconnecting regions of a coating in which the particles are suspended. The continuous coating does not necessarily have to enclose the entire surface area of the underlying core. Openings or other designs can be provided in the continuous coating in known fashion that expose the surface of the otherwise underlying core.

In certain embodiments, the shell comprises a first shell portion and a second shell portion that are compositionally different. In one embodiment, a first shell portion comprises the composition or edible matrix of the invention, and a second shell portion is compositionally different from the first shell portion. In such embodiments the portions may contain different materials, such as for example, polymers, active ingredients, sugars, sugar alcohols, plasticizers, surfactants, dyes, colorants, light diffractive flakes, and flavors.

As used herein, the term "compositionally different" means having features that are readily distinguishable by qualitative or quantitative chemical analysis, physical testing, or visual observation. For example, the first and second shell portions may contain different ingredients, or different levels of the same ingredients, or the first and second shell portions may have different physical or chemical properties, different functional properties, or be visually distinct. Examples of physical or chemical properties that may be different include hydrophylicity, hydrophobicity, hygroscopicity, elasticity, plasticity, tensile strength, crystallinity, and density. Examples of functional properties which may be different include rate and/or extent of dissolution of the material itself or of an active ingredient therefrom, rate of disintegration of the material, permeability to active ingredients, permeability to water or aqueous media, and the like. Examples of visual distinctions include size, shape, topography, or other geometric features, color, hue, opacity, and gloss.

Polymeric materials suitable for use in forming the discontinuous particle phase include edible materials that are solid at a temperature of at least about 37°C. Polymers are low-moisture polymer solutions such as mixtures of gelatin and other hydrocolloids at water contents up to about 30% such as those used to make "gummi" confection forms. In a particularly preferred embodiment, the particle forming polymer is calcium alginate. The calcium ions induce crosslinking reactions that promote particle formation. The particles have a number average particle diameter of at least 100 microns. The particles can on average, when measured using an electronic microscope have a number average particle size less than about 3000 microns, less than about 1000 microns. The particles can be observed as being spherically shaped and having a number average particle size between about 100 microns and about 3000 microns.

In another embodiment, the particles can have a solids content of less than 100%, such as, 75%, 60%, 50%, 40%, 30%, 20%, 10%, 5%, 4%, 3%, 2% and 1%. Notwithstanding the presence of their liquid content, the polymeric particles remain separate and dispersed within the film-forming polymer in its liquid form. The continuous film-forming polymer will also have a solids content. The solids content of the continuous film-forming polymer can be greater than 1%, 5%, 10%, 15%, 20%, 30% or 40%. In one embodiment, polymeric particles for the discontinuous phase have a solids content greater than the solids content of the continuous film-forming polymer, which are combined with one another to form a dispersion that can be used to provide a shell portion over a core having raised particle regions. The raised regions are a result of a greater percentage of water evaporating from the portion with lower percent solids than from the portion with a higher percent solids upon drying. In another embodiment, polymeric particles for the discontinuous have a solids content less than the solids content of the continuous film-forming polymer, which are combined with one another to form a dispersion that can be used to provide a shell portion over a core having depressed particle regions.

Suitable materials for forming the liquid flowable and ultimately continuous phase of dosage form coating, or a portion thereof, include those comprising thermoplastic materials; film formers; thickeners such as gelling polymers or hydrocolloids; low melting hydrophobic materials such as fats and waxes; non-crystallizable carbohydrates; and the like.

Suitable thermoplastic materials can be molded and shaped when heated, and include both water soluble and water insoluble polymers that are generally linear, not crosslinked, nor strongly hydrogen bonded to adjacent polymer chains. Examples of suitable thermoplastic materials include: thermoplastic water swellable cellulose derivatives, thermoplastic water insoluble cellulose derivatives, thermoplastic vinyl polymers, thermoplastic starches, thermoplastic polyalkylene glycols, thermoplastic polyalkylene oxides, and amorphous sugar-glass, and the like, and derivatives, copolymers, and combinations thereof. Examples of suitable thermoplastic water swellable cellulose derivatives include hydroxypropyl cellulose (HPC), hydroxypropylmethyl cellulose (HPMC), methyl cellulose (MC). Examples of suitable thermoplastic water insoluble cellulose derivatives include cellulose acetate (CA), ethyl cellulose (EC), cellulose acetate butyrate (CAB), cellulose propionate. Examples of suitable thermoplastic vinyl polymers include polyvinyl alcohol (PVA) and polyvinyl pyrrolidone (PVP). Examples of suitable thermoplastic starches are disclosed for example in U.S. Patent No. 5,427,614. Examples of suitable thermoplastic polyalkylene glycols include polyethylene glycol. Examples of suitable thermoplastic polyalkylene oxides include polyethylene oxide having a molecular weight from about 100,000 to about 900,000 Daltons. Other suitable thermoplastic materials include sugar in the form on an amorphous glass such as that used to make hard candy forms.

Substantially any film former known in the art is suitable for use in the flowable material of the present invention. Examples of suitable film formers include, but are not limited to, film-forming water soluble polymers, film-forming proteins, film-forming water insoluble polymers, and film-forming pH-dependent polymers. In one embodiment, the film-former for making the core or shell or portion thereof by molding may be selected from cellulose acetate, ammonio methacrylate copolymer type B, shellac, hydroxypropylmethylcellulose, and polyethylene oxide, and combinations thereof.

Suitable film-forming water soluble polymers include water soluble vinyl polymers such as polyvinylalcohol (PVA); water soluble polycarbohydrates such as hydroxypropyl starch, hydroxyethyl starch, pullulan, methylethyl starch, carboxymethyl starch, pre-gelatinized starches, and film-forming modified starches; water swellable cellulose derivatives such as hydroxypropyl cellulose (HPC), hydroxypropylmethyl cellulose (HPMC), methyl cellulose (MC), hydroxyethylmethylcellulose (HEMC), hydroxybutylmethylcellulose (HBMC), hydroxyethylethylcellulose (HEEC), and hydroxyethylhydroxypropylmethyl cellulose (HEMPMC); water soluble copolymers such as methacrylic acid and methacrylate ester copolymers, polyvinyl alcohol and polyethylene glycol copolymers, polyethylene oxide and polyvinylpyrrolidone copolymers; and derivatives and combinations thereof.

Suitable film-forming proteins may be natural or chemically modified, and include gelatin, whey protein, myofibrillar proteins, coagulatable proteins such as albumin, casein, caseinates and casein isolates, soy protein and soy protein isolates, zein;; and polymers, derivatives and mixtures thereof.

Suitable film-forming water insoluble polymers, include for example ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof.

Suitable film-forming pH-dependent polymers include enteric cellulose derivatives, such as for example hydroxypropyl methylcellulose phthalate, hydroxypropyl methylcellulose acetate succinate, cellulose acetate phthalate; natural resins, such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename, EUDRAGIT S, and poly(methacrylic acid, methyl methacrylate) 1:1, which is commercially available from Rohm Pharma GmbH under the tradename, EUDRAGIT L, and the like, and derivatives, salts, copolymers, and combinations thereof.

One suitable hydroxypropylmethylcellulose compound for use as a thermoplastic film-forming water soluble polymer is "HPMC 2910", which is a cellulose ether having a degree of substitution of about 1.9 and a hydroxypropyl molar substitution of 0.23, and containing, based upon the total weight of the compound, from about 29% to about 30% methoxyl groups and from about 7% to about 12% hydroxylpropyl groups. HPMC 2910 is commercially available from the Dow Chemical Company under the tradename METHOCEL E. METHOCEL E5, which is one grade of HPMC-291 0 suitable for use in the present invention, has a viscosity of about 4 to 6 cps (4 to 6 millipascal-seconds) at 20°C in a 2% aqueous solution as determined by a Ubbelohde viscometer. Similarly, METHOCEL E6, which is another grade of HPMC-2910 suitable for use in the present invention, has a viscosity of about 5 to 7 cps (5 to 7 millipascal-seconds) at 20°C in a 2% aqueous solution as determined by a Ubbelohde viscometer. METHOCEL E 15, which is another grade of HPMC-2910 suitable for use in the present invention, has a viscosity of about 15000 cps (15 millipascal-seconds) at 20°C in a 2% aqueous solution as determined by a Ubbelohde viscometer. As used herein, "degree of substitution" shall mean the average number of substituent groups attached to a anhydroglucose ring, and "hydroxypropyl molar substitution" shall mean the number of moles of hydroxypropyl per mole anhydroglucose.

One suitable polyvinyl alcohol and polyethylene glycol copolymer is commercially available from BASF Corporation under the tradename KOLLICOAT IR.

As used herein, "modified starches" include starches that have been modified by crosslinking, chemically modified for improved stability or optimized performance, or physically modified for improved solubility properties or optimized performance. Examples of chemically-modified starches are well known in the art and typically include those starches that have been chemically treated to cause replacement of some of its hydroxyl groups with either ester or ether groups. Crosslinking, as used herein, may occur in modified starches when two hydroxyl groups on neighboring starch molecules are chemically linked. As used herein, "pre-gelatinized starches" or "instantized starches" refers to modified starches that have been pre-wetted, then dried to enhance their coldwater solubility. Suitable modified starches are commercially available from several suppliers such as, for example, A.E. Staley Manufacturing Company, and National Starch & Chemical Company. One suitable film forming modified starch includes the pre-gelatinized waxy maize derivative starches that are commercially available from National Starch & Chemical Company under the tradenames PURITY GUM and FILMSET, and derivatives, copolymers, and mixtures thereof. Such waxy maize starches typically contain, based upon the total weight of the starch, from about 0 percent to about 18 percent of amylose and from about 100% to about 88% of amylopectin.

Another suitable film forming modified starch includes the hydroxypropylated starches, in which some of the hydroxyl groups of the starch have been etherified with hydroxypropyl groups, usually via treatment with propylene oxide. One example of a suitable hydroxypropyl starch that possesses film-forming properties is available from Grain Processing Company under the tradename, PURE-COTE B790.

Suitable tapioca dextrins for use as film formers include those available from National Starch & Chemical Company under the tradenames CRYSTAL GUM or K-4484, and derivatives thereof such as modified food starch derived from tapioca, which is available from National Starch and Chemical under the tradename PURITY GUM 40, and copolymers and mixtures thereof.

Any thickener known in the art is suitable for use in the flowable material of the continuous phase or the solids of the discontinuous phase of the present invention. Examples of such thickeners include but are not limited to hydrocolloids (also referred to herein as gelling polymers), clays, gelling starches, and crystallizable carbohydrates, and derivatives, copolymers and mixtures thereof.

Examples of suitable hydrocolloids are alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, pectin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, and derivatives and mixtures thereof. Additional suitable thickening hydrocolloids include low-moisture polymer solutions such as mixtures of gelatin and other hydrocolloids at water contents up to about 30%, such as for example those used to make "gummi" confection forms.

Additional suitable thickeners include crystallizable carbohydrates, and the like, and derivatives and combinations thereof. Suitable crystallizable carbohydrates include the monosaccharides and the oligosaccharides. Of the monosaccharides, the aldohexoses e.g., the D and L isomers of allose, altrose, glucose, mannose, gulose, idose, galactose, talose, and the ketohexoses e.g., the D and L isomers of fructose and sorbose along with their hydrogenated analogs: e.g., glucitol (sorbitol), and mannitol are preferred. Of the oligosaccharides, the 1,2-disaccharides sucrose and trehalose, the 1,4-disaccharides maltose, lactose, and cellobiose, and the 1,6-disaccharides gentiobiose and melibiose, as well as the trisaccharide raffinose are preferred along with the isomerized form of sucrose known as isomaltulose and its hydrogenated analog isomalt. Other hydrogenated forms of reducing disaccharides (such as maltose and lactose), for example, maltitol and lactitol are also preferred. Additionally, the hydrogenated forms of the aldopentoses: e.g., D and L ribose, arabinose, xylose, and lyxose and the hydrogenated forms of the aldotetroses: e.g., D and L erythrose and threose are preferred and are exemplified by xylitol and erythritol, respectively.

Any plasticizer known in the pharmaceutical art is suitable for use in the composition of the continuous phase or in the solid particles of the discontinuous phase, and may include, but not be limited to polyethylene glycol; glycerin; sorbitol; triethyl citrate; tribuyl citrate; dibutyl sebecate; vegetable oils such as castor oil; surfactants such as polysorbates, sodium lauryl sulfates, and dioctyl-sodium sulfosuccinates; propylene glycol; mono acetate of glycerol; diacetate of glycerol; triacetate of glycerol; natural gums and mixtures thereof. In solutions containing a cellulose ether film former, an optional plasticizer may be present in an amount, based upon the total weight of the solution, from about 0% to about 40%.

In one embodiment of the invention, the liquid flowable material for forming the continuous phase portion of the coating comprises gelatin as a gelling polymer. Gelatin is a natural, thermogelling polymer. It is a tasteless and colorless mixture of derived proteins of the albuminous class which is ordinarily soluble in warm water. Two types of gelatin - Type A and Type B - are commonly used. Type A gelatin is a derivative of acid-treated raw materials. Type B gelatin is a derivative of alkali-treated raw materials. The moisture content of gelatin, as well as its Bloom strength, composition and original gelatin processing conditions, determine its transition temperature between liquid and solid. Bloom is a standard measure of the strength of a gelatin gel, and is roughly correlated with molecular weight. Bloom is defined as the weight in grams required to move a half-inch diameter plastic plunger 4 mm into a 6.67% gelatin gel that has been held at 10°C for 17 hours. In a preferred embodiment, the flowable material is an aqueous solution comprising 20% 275 Bloom pork skin gelatin, 20% 250 Bloom Bone Gelatin, and approximately 60% water.

Suitable xanthan gums include those available from C.P. Kelco Company under the tradenames KELTROL 1000, XANTROL 180, or K9B310.

Suitable clays include smectites such as bentonite, kaolin, and laponite; magnesium trisilicate, magnesium aluminum silicate, and the like, and derivatives and mixtures thereof.

"Acid-hydrolyzed starch," as used herein, is one type of modified starch that results from treating a starch suspension with dilute acid at a temperature below the gelatinization point of the starch. During the acid hydrolysis, the granular form of the starch is maintained in the starch suspension, and the hydrolysis reaction is ended by neutralization, filtration and drying once the desired degree of hydrolysis is reached. As a result, the average molecular size of the starch polymers is reduced. Acid-hydrolyzed starches (also known as "thin boiling starches") tend to have a much lower hot viscosity than the same native starch as well as a strong tendency to gel when cooled.

"Gelling starches," as used herein, include those starches that, when combined with water and heated to a temperature sufficient to form a solution, thereafter form a gel upon cooling to a temperature below the gelation point of the starch. Examples of gelling starches include, but are not limited to, acid hydrolyzed starches such as that available from Grain Processing Corporation under the tradename PURE-SET B950; hydroxypropyl distarch phosphate such as that available from Grain Processing Corporation under the tradename, PURE-GEL B990, and mixtures thereof.

Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

Suitable non-crystallizable carbohydrates include non-crystallizable sugars such as polydextrose, and starch hydrolysates, e.g. glucose syrup, corn syrup, and high fructose corn syrup; and non-crystallizable sugar-alcohols such as maltitol syrup.

In certain embodiments, at least one coating of the shell or shell portions is in direct contact with the core. In certain other embodiments, the inner coating of the shell or shell portions is in direct contact with a subcoating that substantially surrounds the core. In certain embodiments, the shell or a shell portion may comprise one ore more openings therein.

In embodiments in which one or more coatings for the shell or shell portion is applied to the core by molding, at least a portion of the shell surrounds the core such that the shell inner surface resides substantially conformally upon the core outer surface. As used herein, the term "substantially conformally" shall mean that the inner surface of the shell has peaks and valleys or indentations and protrusions corresponding substantially inversely to the peaks and valleys of the outer surface of the core.

In one embodiment, the dosage form of the invention comprises: a) a core containing an active ingredient; b) an optional subcoating that substantially covers the core; and c) a coating that comprises at least first and second shell portions residing on the surface of the subcoating. The term "shell portion" should not be construed to require a physical or compositional difference in matter. Rather, a shell portion could be part of one and the same continuous shell section but distinguished from another portion by reference to the region of the core in contact or some other similar distinguishing characteristic. As used herein, "substantially covers" shall mean at least about 95 percent of the surface area of the core is covered by the subcoating. The use of subcoatings is well known in the art and disclosed in, for example, United States Patent Nos. 3,185,626.

In one preferred embodiment, the discontinuous portion of the shell facilitates more rapid disintegration of the dosage form. The integrity of the shell is dependent on the uniformity of the film in the shell. The discontinuous portion creates spaces in the film which disrupt this uniformity. When the coated dosage form is placed in a liquid medium, the continuous portions of the shell will disintegrate more rapidly since the film is not uniform and is disrupted more easily.

In one embodiment, the dosage form may contain, based upon the total dry weight of the dosage form, from about 1 percent to about 99 percent, e.g. from about 1 to about 50 percent of the shell comprising the continuous portion, and from about 0.1 percent to about 75, e.g. from about 0.1 percent to about 40 percent of the discontinuous portion. The shell may contain, based upon the total weight of the shell, from about 40 percent to about 99 percent, e.g. from about 40 percent to about 80 percent of the continuous portion, and from about 1 percent to about 50 percent, e.g. from about 1 percent to about 40 percent, of the discontinuous portion.

In one embodiment the discontinuous phase is present in the non-crystalline or amorphous phase, both in the liquid coating composition and the solid dried dosage form. In this embodiment the discontinuous phase is substantially free of crystalline materials.

In one embodiment, a dispersion of the composition comprising the continuous film-forming polymer and a polymeric particle for the discontinuous phase is used to prepare the shell. In particular, polymeric particles and liquid gelling polymer are dispersed in an aqueous solution. The dispersion is applied to a core, by for example molding, dipping, enrobing, or other means. Preferably, the dispersion is applied to the core by molding. After application of the dispersion to the core, the core is cooled, preferably at a relatively high temperature.

Injection molding is performed by injecting the dispersion into a heated molding chamber such as, for example, the system described in U.S. Patent No. 6,326,026. In this embodiment, the dispersion comprises the polymeric particle dispersed in a liquid carrier comprising the gelling polymer. The dispersion is cooled and solidifies in the molding chamber into a shaped form (i.e., having the shape of the mold). Still further, the inner surface of the mold defining the cavity can be provided with channels that guide and cause the particles in the dispersion to position themselves in predetermined patterns over the core surface. In alternate embodiments the shell is applied via processes known in the art such as dipping as disclosed in for example, U.S. Patent No. 4,820,524; or enrobing as disclosed in for example, U.S. Patent Nos. 6,482,516 and 5,146,730 using a pre-made film.

The dispersion for making the shell by injection molding may optionally comprise adjuvants or excipients, which may comprise up to about 30% by weight of the dispersion. Examples of suitable adjuvants or excipients include detackifiers, humectants, surfactants, anti-foaming agents, colorants, flavorants, sweeteners, opacifiers, and the like.

In one embodiment, prior to injection molding onto a core, the discontinuous phase material is held in one reservoir and the continuous phase material is held in a separate reservoir. The materials are mixed in-line during the injection process through the use of an in-line static mixer. The level of mixing is controlled via the speed of the static mixer, which is dependent on the flow rate of the two materials into the mixer. The rate at which the materials are injected is dependent on the level of positive air pressure which is attached to each reservoir. The level of air pressure can range from about 10 pounds per square inch (psi) to about 100 psi.

In another embodiment the two materials are mixed in a common solution and held in a single reservoir prior to application to the core.

In embodiments in which the coatings are prepared by molding, the continuous regions of the coatings typically are substantially free of pores in the diameter range of 0.5 to 5.0 microns, i.e. has a pore volume in the pore diameter range of 0.5 to 5.0 microns of less than about 0.02 cc/g, preferably less than about 0.01 cc/g, more preferably less than about 0.005 cc/g. Typical compressed materials have pore volumes in this diameter range of more than about 0.02 cc/g. Pore volume, pore diameter and density may be determined using a Quantachrome Instruments PoreMaster 60 mercury intrusion porosimeter and associated computer software program known as "Porowin." The procedure is documented in the Quantachrome Instruments PoreMaster Operation Manual. The PoreMaster determines both pore volume and pore diameter of a solid or powder by forced intrusion of a non-wetting liquid (mercury), which involves evacuation of the sample in a sample cell (penetrometer), filling the cell with mercury to surround the sample with mercury, applying pressure to the sample cell by: (i) compressed air (up to 50 psi maximum); and (ii) a hydraulic (oil) pressure generator (up to 60000 psi maximum). Intruded volume is measured by a change in the capacitance as mercury moves from outside the sample into its pores under applied pressure. The corresponding pore size diameter (d) at which the intrusion takes place is calculated directly from the so-called "Washburn Equation": d= -(4γ(cosθ)/P) where γ is the surface tension of liquid mercury, θ is the contact angle between mercury and the sample surface and P is the applied pressure.

Suitable solvents for optional use as components of the flowable material for making the shell, or a portion thereof by molding include water; polar organic solvents such as methanol, ethanol, isopropanol, acetone, and the like; and non-polar organic solvents such as methylene chloride, and the like; and mixtures thereof.

The shell portions of the present invention may be prepared by molding, using a solvent-free process, or a solvent-based process, and depending on the method used, typically comprise a variety of excipients which are useful for conferring desired properties to the shell portions. The shell portions may optionally further comprise one or more active ingredients.

In one embodiment, either the continuous portion or the discontinuous portion may optionally comprise a flavoring agent or sensate. As used herein, a "sensate" is a chemical agent that elicits a sensory effect in the mouth, nose, and/or throat other than aroma or flavor. Examples of such sensory effects include, but are not limited to, cooling, warming, tingling, mouth watering (succulent), astringent, and the like. Sensate agents suitable for use in the present invention are commercially available and may be purchased from, for example, International Flavor & Fragrances.

Typical each coating in the shell layer can have individual thicknesses in the range of about 50 to about 4000 microns. In certain preferred embodiments, at least one coating in the shell layer has a thickness of less than 800 microns. In embodiments wherein at least one coating for the shell layer (or part thereof) is prepared by a solvent-free molding process, such shell layer (or part thereof) typically has a thickness of about 500 to about 4000 microns, e.g. about 500 to about 2000 microns, say about 500 to about 800 microns, or about 800 to about 1200 microns. In embodiments wherein the shell layer (or part thereof) is prepared by a solvent-based molding process, the shell layer typically has a thickness of less than about 800 microns, e.g. about 100 to about 600 microns, say about 150 to about 400 microns.

In other particular embodiments the thickness will vary between the continuous and discontinuous regions of the shell, in that the average thickness of one region is at least 10% greater than the smallest or greatest thickness of the other region. In other words, in one embodiment wherein the incorporation of a discontinuous phase produces a coating having a concave dimpled appearance, the average thickness of the continuous phase will be at least 10% greater than average smallest thickness as measured from the base of each concave dimple. In an embodiment wherein the incorporation of a discontinuous phase produces a coating having a raised dimpled appearance, the average thickness of the continuous phase will be at least 10% less than average greatest thickness as measured from the top of each raised dimple. In one embodiment the dimpled portions of the film in the embodiment with the concave dimpled appearance have a diameter between about 30 microns to 3000 microns, or about 100 microns to 1000 microns, or about 300 microns to about 1000 microns. In another embodiment the dimpled portions of the film in the embodiment with the raised dimpled appearance have a diameter between about 30 microns to 3000 microns, or about 100 microns to 1000 microns, or about 300 microns to about 1000 microns.

In another embodiment the continuous phase and discontinuous phase contain the same level of dissolved solids in the liquid dispersion phase prior to application as a shell on a dosage form. In this embodiment, the resulting film will be substantially smooth upon drying, without raised or dimpled portions. The continuous phase and discontinuous phases will still be compositionally different.

The following non-limiting examples are provided to further illustrate the inventions described herein.

Part A: Preparation of Gelatin-Based Coating Solution Containing Alginate Micro-beads

### Preparation of alginate-based micro-beads

The micro-beads are prepared by gradually dropping 20 ml of the alginate solution (1.5%, Kelton HV from CP Kelco) into a 300 ml of calcium chloride solution (0.5%) with agitation while mixing with magnetic stirring bar, using a dropper, and a Novamatrix^{®} electrostatic bead generator. The resulting spherical gel beads are then filtered through a 100-mesh screen and washed with the purified water.

### Preparation of gelatin-based coating solution containing alginate beads

The gelatin solution is prepared by adding 70 g of 275-bloom pork skin gelatin into a pre-heated 130 g of purified water at 55°C with agitation at 100 RPM. The entire alginate beads made from the first step are then added into the gelatin solution while mixing at 100 RPM.

### Part B (Reference example): Preparation of Gelatin-Based Coating Solution Containing wax-based micro-beads

Preparation of waxed based beads. The micro-beads are prepared by pouring a wax composition consisting of: 16.25 g of Castrowax MP 80 (Supplier is Caschem) and 0.029 g of D&C yellow dye #11 (Supplier is Sensient, Inc.) pre-melted at 90 °C, into a vessel containing 210g of purified water with Gelucire 50/13 as a dispersant, pre-heated at 85°C. The mixture is then vigorously agitated at 800 rpm for 2 minutes before cooling. During the cooling process, the dispersed oily droplets are solidified. The waxy beads are collected by filtration and washed with cold purified water.

### Preparation of gelatin-based Coating Solution containing wax beads

The gelatin solution is prepared by adding 105 g of 250-bloom bone gelatin into a pre-heated 195 g of purified water at 55°C with agitation at 100 RPM. All of the wax beads made from the first step are then blended into the gelatin solution.

### Part C: Compressed tablets

The following ingredients are mixed well in a plastic bag end-over-end for approximately 3 minutes: 89.4 parts acetaminophen USP (590 mg/tablet) and 8.0 parts of synthetic wax X-2068 T20 (53 mg/tablet). Next, 2.1 parts of sodium starch glycolate (EXPLOTAB) (13.9 mg/tablet) and 0.09 parts of silicon dioxide (0.6 mg/tablet) are added to the bag, and mixed well end-over-end for approximately 3 minutes. Then 0.36 parts of magnesium stearate NF (2.4 mg/tablet) are added to the bag, and the ingredients are again mixed end-over-end for approximately 1 minute. The resulting dry blend is compressed into tablets on a rotary tablet compression module using 7/16 inch extra deep concave tablet tooling. The compression module is a double row, rotary apparatus, comprising a fill zone, insertion zone, compression zone, ejection zone, and purge zone. The dies of the compression module are filled using vacuum assistance, with mesh screen filters located in die wall ports of each die. The resulting tablets (cores) have an average weight of 660 mg, thickness of 0.306 inches, and hardness of 3.2 kp.

### Part D: Tablet Coating

The tablets from Part B are conveyed to a molding module via a transfer device. A portion of the tablets are coated with gelatin-based solutions containing alginate beads from Part A and a portion of the tablets are coated with the gelatin-based solutions containing wax based beads from Part B to form a shell. A molding module applies the shell to the tablets. Tablets are transferred to the mold assemblies, which then close over the tablets. Flowable material, which is heated to a flowable state in a reservoir, is injected into the mold cavities created by the closed mold assemblies. The temperature of the shell flowable material is then decreased, hardening it. The mold assemblies open and eject the coated cores. Coating is performed in two steps, each half of the tablets being coated separately.

## Claims

1. A liquid pharmaceutical dosage coating composition comprising:
a) a shell-forming component comprising a dispersion of:
i) at least one film-forming polymer in liquid form that is liquid at temperatures of 37°C or above; and
ii) at least one particle forming polymer in solid id particle form for a discontinuous phase that is dispersed in said polymer in liquid form in (i),
wherein the solid particles have a number average particle size of at least 100 microns and wherein the solid particles are a low-moisture hydrocolloid solution with a water content up to 30%.

2. A liquid pharmaceutical dosage coating composition according to claim 1 wherein the at least one film-forming polymer is water-insoluble.

3. A liquid pharmaceutical dosage: coating composition according to claim 1 wherein the at least one film-forming polymer is a water-soluble or water swellable gelling polymer.

4. A liquid pharmaceutical dosage coaling composition according to claim 3 wherein the gelling polymer consists essentially of at least one gelatin.

5. A liquid pharmaceutical dosage coating composition according to claim 2 wherein the water-insoluble film-forming polymer is crosslinked.

6. A liquid pharmaceutical dosage coating composition according to claim 1 wherein the at least one particle forming polymer is water-insoluble.

7. A liquid pharmaceutical dosage costing composition according to claim 6 wherein the water-insoluble particle forming particle is crosslinked.

8. A liquid pharmaceutical dosage coating composition according to claim 1 wherein the particles have a number average particle size less than about 3000 microns, preferably less than about 1000 microns.

9. A liquid pharmaceutical dosage coating composition according to claim 8 wherein the particles are spherically shaped and have a number average particle size between about 30 microns to about 3000 microns.

10. The liquid pharmaceutical dosage coating composition according to claim 1 wherein the solid particles are amorphous.

11. The liquid pharmaceutical dosage coating composition according to claim 1 wherein the solid particles are water soluble.

12. A solid pharmaceutical dosage form comprising a core and at least one coating layer, which is formed from the liquid pharmaceutical dosage costing composition according to claim 1, that comprises a solid continuous film having discrete compositionally different regions dispersed therein.

13. A solid pharmaceutical dosage form according to claim 12 wherein the core comprises a compressed powder tablet.

14. A slid pharmaceutical dosage form according to claim 12 wherein at least one coating layer has a concave dimpled surface area comprising dimpled portions.

15. A solid pharmaceutical dosage form according to claim 14 wherein the diameter of dimpled portions in said concave dimpled surface is between about 100 microns to about 3000 microns.

16. A solid pharmaceutical dosage form according to claim 14 wherein the thickness of the solid continuous film is, on average, at least 10% greater than the smallest thickness of the solid continuous film and the overlying discrete compositionally different regions in said regions having a concave surface area.

17. A solid pharmaceutical dosage form according to claim 12 wherein at least one coating layer has a raised dimple surface area comprising dimpled portions.

18. A solid pharmaceutical dosage form according to claim 17 wherein the diameter of dimpled portions in said raised dimpled surface area is between about 100 microns to about 3000 microns.

19. A solid pharmaceutical dosage form according to claim 17 wherein the thickness of the solid continuous film is, on average, at least 10% less than the greatest thickness of the solid continuous film and overlying discrete compositionally different region in said regions having raised dimple surface area.

20. A solid pharmaceutical dosage form according to claim 12 wherein the discrete compositionally different regions have greater water solubility than the solid continuous film.

21. A process for preparing a solid dosage form comprising coating a core containing a pharmaceutical active ingredient with the composition according to claim 1.

22. A process for preparing a solid dosage form according to claim 21 wherein the solid particles are sufficiently hydrated to deform in an injection molding system.

23. A process for preparing a core and shell dosage form comprising:
a) forming a compressed core containing at least one pharmaceutical active ingredient in compression tableting machine; and
b) coating the compressed core with the composition according to claim 1.

24. A process for preparing a core and shell dosage form comprising
a) forming a solid, compressed core containing at least one pharmaceutical active ingredient in a tableting machine;
b) introducing the compressed core into a mold cavity; and
c) injecting the composition according to claim 1 into the mold cavity to coat at least a portion of the compressed core;
and optionally comprising the steps of
d) rotating the mold cavity; and
e) injecting a liquid curable composition into said mold to coat at least a second portion of the compressed core.

## Patentansprüche

1. Flüssige pharmazeutische Dosierungsüberzugszusammensetzung, umfassend:
a) eine mantelbildende Komponente, die eine Dispersion aus:
i) wenigstens einem filmbildenden Polymer in flüssiger Form, das bei Temperaturen von 37°C oder darüber flüssig ist; und
ii) wenigstens einem teilchenbildenden Polymer in fester Teilchenform für eine diskontinuierliche Phase, die in dem Polymer in flüssiger Form in (i) dispergiert ist,
umfasst,
wobei die festen Teilchen eine Teilchengröße im Zahlenmittel von wenigstens 100 Mikrons besitzen und wobei die festen Teilchen eine Hydrokolloidlösung mit niedrigem Feuchtegehalt mit einem Wassergehalt bis zu 30% sind.

2. Flüssige pharmazeutische Dosierungsüberzugszusammensetzung nach Anspruch 1, wobei wenigstens ein filmbildendes Polymer wasserunlöslich ist.

3. Flüssige pharmazeutische Dosierungsüberzugszusammensetzung nach Anspruch 1, wobei das wenigstens eine filmbildende Polymer ein wasserlösliches oder wasserquellbares gelierendes Polymer ist.

4. Flüssige pharmazeutische Dosierungsüberzugszusammensetzung nach Anspruch 3, wobei das gelierende Polymer im Wesentlichen aus wenigstens einer Gelatine besteht.

5. Flüssige pharmazeutische Dosierungsüberzugszusammensetzung nach Anspruch 2, wobei das wasserunlösliche filmbildende Polymer vernetzt ist.

6. Flüssige pharmazeutische Dosierungsüberzugszusammensetzung nach Anspruch 1, wobei das wenigstens eine teilchenbildende Polymer wasserunlöslich ist.

7. Flüssige pharmazeutische Dosierungsüberzugszusammensetzung nach Anspruch 6, wobei das wasserunlösliche teilchenbildende Teilchen vernetzt ist.

8. Flüssige pharmazeutische Dosierungsüberzugszusammensetzung nach Anspruch 1, wobei die Teilchen eine Teilchengröße im Zahlenmittel von weniger als etwa 3000 Mikrons, vorzugsweise weniger als etwa 1000 Mikrons besitzen.

9. Flüssige pharmazeutische Dosierungsüberzugszusammensetzung nach Anspruch 8, wobei die Teilchen kugelförmig ausgebildet sind und eine Teilchengröße im Zahlenmittel zwischen etwa 30 Mikrons bis etwa 3000 Mikrons besitzen.

10. Flüssige pharmazeutische Dosierungsüberzugszusammensetzung nach Anspruch 1, wobei die festen Teilchen amorph sind.

11. Flüssige pharmazeutische Dosierungsüberzugszusammensetzung nach Anspruch 1, wobei die festen Teilchen wasserlöslich sind.

12. Feste pharmazeutische Dosierungsform, die einen Kern und wenigstens eine Überzugsschicht umfasst, die aus der flüssigen pharmazeutischen Dosierungsüberzugszusammensetzung nach Anspruch 1 gebildet ist, die einen festen kontinuierlichen Film mit diskreten zusammensetzungsmäßig unterschiedlichen Bereichen, die darin dispergiert sind, umfasst.

13. Feste pharmazeutische Dosierungsform nach Anspruch 12, wobei der Kern eine verpresste Pulvertablette umfasst.

14. Feste pharmazeutische Dosierungsform nach Anspruch 12, wobei wenigstens eine Überzugsschicht eine konkave eingedrückte Oberfläche besitzt, die eingedrückte Abschnitte umfasst.

15. Feste pharmazeutische Dosierungsform nach Anspruch 14, wobei der Durchmesser der eingedrückten Abschnitte in der konkaven eingedrückten Oberfläche zwischen etwa 100 Mikrons bis etwa 3000 Mikrons beträgt.

16. Feste pharmazeutische Dosierungsform nach Anspruch 14, wobei die Dicke des festen kontinuierlichen Films, durchschnittlich, wenigstens 10% größer ist als die geringste Dicke des festen kontinuierlichen Films und der darüberliegenden diskreten zusammensetzungsmäßig unterschiedlichen Bereiche in den Bereichen mit konkaven Oberflächen.

17. Feste pharmazeutische Dosierungsform nach Anspruch 12, wobei wenigstens eine Überzugsschicht eine erhabene eingedrückte Oberfläche besitzt, die eingedrückte Abschnitte umfasst.

18. Feste pharmazeutische Dosierungsform nach Anspruch 17, wobei der Durchmesser der eingedrückten Abschnitte in der erhabenen eingedrückten Oberfläche zwischen etwa 100 Mikrons bis etwa 3000 Mikrons beträgt.

19. Feste pharmazeutische Dosierungsform nach Anspruch 17, wobei die Dicke des festen kontinuierlichen Films, durchschnittlich, wenigstens 10% geringer ist als die größte Dicke des festen kontinuierlichen Films und darüberliegenden diskreten zusammensetzungsmäßig unterschiedlichen Bereichs in den Bereichen mit erhabener eingedrückter Oberfläche.

20. Feste pharmazeutische Dosierungsform nach Anspruch 12, wobei die diskreten zusammensetzungsmäßig unterschiedlichen Bereiche höhere Wasserlöslichkeit besitzen als der feste kontinuierliche Film.

21. Verfahren zur Herstellung einer festen Dosierungsform, welches umfasst, dass ein Kern, der einen pharmazeutisch aktiven Inhaltsstoff enthält, mit der Zusammensetzung nach Anspruch 1 überzogen wird.

22. Verfahren zur Herstellung einer festen Dosierungsform nach Anspruch 21, wobei die festen Teilchen ausreichend hydratisiert werden, um sich in einem Spritzgusssystem zu verformen.

23. Verfahren zur Herstellung einer Kern-Mantel-Dosierungsform, umfassend:
a) Ausbilden eines verpressten Kerns, der wenigstens einen pharmazeutischen aktiven Inhaltstoff enthält, in einer Verpressungstablettiermaschine; und
b) Überziehen des verpressten Kerns mit der Zusammensetzung nach Anspruch 1.

24. Verfahren zur Herstellung einer Kern-Mantel-Dosierungsform, umfassend:
a) Ausbilden eines festen, verpressten Kerns, der wenigstens einen pharmazeutischen aktiven Inhaltsstoff enthält, in einer Tablettiermaschine;
b) Einbringen des verpressten Kerns in einen Formhohlraum; und
c) Einspritzen der Zusammensetzung nach Anspruch 1 in den Formhohlraum, um wenigstens einen Abschnitt des verpressten Kerns zu überziehen;
und gegebenenfalls die Schritte umfassend
d) Drehen des Formhohlraums; und
e) Einspritzen einer flüssigen aushärtbaren Zusammensetzung in die Form, um wenigstens einen zweiten Abschnitt des verpressten Kerns zu überziehen.

## Revendications

1. Composition d'enrobage liquide pour forme pharmaceutique comprenant :
a) un composant formant coque comprenant une dispersion de :
i) au moins un polymère filmogène sous forme liquide qui est liquide à des températures de 37°C ou plus ; et
ii) au moins un polymère formant particules sous forme de particules solides, destiné à former une phase discontinue qui est dispersée dans ledit polymère sous forme liquide en (i),
dans laquelle les particules solides ont une taille de particule moyenne en nombre d'au moins 100 microns et dans laquelle les particules solides sont une solution hydrocolloidale à basse teneur d'humidité ayant une teneur d'eau maximale de 30 %.

2. Composition d'enrobage liquide pour forme pharmaceutique selon la revendication 1, dans laquelle ledit au moins un polymère filmogène est insoluble dans l'eau.

3. Composition d'enrobage liquide pour forme pharmaceutique selon la revendication 1, dans laquelle ledit au moins un polymère filmogène est un polymère soluble dans l'eau ou du type gélifiant pouvant gonfler dans l'eau.

4. Composition d'enrobage liquide pour forme pharmaceutique selon la revendication 3, dans laquelle le polymère gélifiant se compose essentiellement d'au moins une gélatine.

5. Composition d'enrobage liquide pour forme pharmaceutique selon la revendication 2, dans laquelle le polymère filmogène insoluble dans l'eau est réticulé.

6. Composition d'enrobage liquide pour forme pharmaceutique selon la revendication 1, dans laquelle ledit au moins polymère formant particules est insoluble dans l'eau.

7. Composition d'enrobage liquide pour forme pharmaceutique selon la revendication 6, dans laquelle le polymère formant particules insoluble dans l'eau est réticulé.

8. Composition d'enrobage liquide pour forme pharmaceutique selon la revendication 1, dans laquelle les particules ont une taille de particule moyenne en nombre inférieure à environ 3 000 microns, de préférence, inférieure à environ 1 000 microns.

9. Composition d'enrobage liquide pour forme pharmaceutique selon la revendication 8, dans laquelle les particules sont de forme sphérique et ont une taille de particule moyenne en nombre comprise entre environ 30 et environ 3 000 microns.

10. Composition d'enrobage liquide pour forme pharmaceutique selon la revendication 1, dans laquelle les particules solides sont amorphes.

11. Composition d'enrobage liquide pour forme pharmaceutique selon la revendication 1, dans laquelle les particules solides sont solubles dans l'eau.

12. Forme pharmaceutique solide comprenant un coeur et au moins une couche d'enrobage, qui est formée à partir de la composition d'enrobage liquide pour forme pharmaceutique selon la revendication 1, qui comprend un film continu solide comportant des régions individuelles de composition différente dispersées dans celui-ci.

13. Forme pharmaceutique solide selon la revendication 12, dans laquelle le coeur comprend un comprimé du type poudre comprimée.

14. Forme pharmaceutique solide selon la revendication 12, dans laquelle au moins une couche d'enrobage présente une surface ondulée concave comprenant des parties ondulées.

15. Forme pharmaceutique solide selon la revendication 14, dans laquelle le diamètre des parties ondulées dans ladite surface ondulée concave est compris entre environ 100 et environ 3 000 microns.

16. Forme pharmaceutique solide selon la revendication 14, dans laquelle l'épaisseur du film continu solide est, en moyenne, au moins 10 % supérieure à la plus petite épaisseur du film continu solide et des régions individuelles couvrantes de composition différente dans lesdites régions présentant une surface concave.

17. Forme pharmaceutique solide selon la revendication 12, dans laquelle au moins une couche d'enrobage présente une surface ondulée surélevée comprenant des parties ondulées.

18. Forme pharmaceutique solide selon la revendication 17, dans laquelle le diamètre des parties ondulées dans ladite surface ondulée surélevée est compris entre environ 100 et environ 3 000 microns.

19. Forme pharmaceutique solide selon la revendication 17, dans laquelle l'épaisseur du film continu solide est, en moyenne, au moins 10 % inférieure à la plus grande épaisseur du film continu solide et des régions individuelles couvrantes de composition différente dans lesdites régions présentant une surface ondulée surélevée.

20. Forme pharmaceutique solide selon la revendication 12, dans laquelle les régions individuelles de composition différente ont une solubilité dans l'eau supérieure à celle du film continu solide.

21. Procédé de préparation d'une forme pharmaceutique solide comprenant l'enrobage d'un coeur contenant un principe pharmaceutique actif avec la composition selon la revendication 1.

22. Procédé de préparation d'une forme pharmaceutique solide selon la revendication 21, dans lequel les particules solides sont suffisamment hydratées pour se déformer dans un système de moulage par injection.

23. Procédé de préparation d'une forme pharmaceutique du type coeur et coque comprenant :
a) la formation par compression d'un coeur contenant au moins un principe pharmaceutique actif dans une presse à comprimés ; et
b) l'enrobage du coeur comprimé avec la composition selon la revendication 1.

24. Procédé de préparation d'une forme pharmaceutique du type coeur et coque comprenant :
a) la formation par compression d'un coeur solide contenant au moins un principe pharmaceutique actif dans une presse à comprimés ;
b) l'introduction du coeur comprimé dans une cavité de moule ; et
c) l'injection de la composition selon la revendication 1 dans la cavité de moule pour enrober au moins une partie du coeur comprimé ;
et comprenant éventuellement les étapes suivante
d) la mise en rotation de la cavité de moule ; et
e) l'injection d'une composition durcissable liquide dans ledit moule pour enrober au moins une seconde partie du coeur comprimé.
